(19) **Europäisches Patentamt / European Patent Office / Office européen des brevets**

(11) **EP 2 725 354 B1**

(12) # EUROPEAN PATENT SPECIFICATION

(45) Date of publication and mention of the grant of the patent:
**09.05.2018 Bulletin 2018/19**

(51) Int Cl.:
*G01N 33/22* (2006.01)    *F23N 5/00* (2006.01)
*G05B 13/02* (2006.01)

(21) Application number: **12189579.1**

(22) Date of filing: **23.10.2012**

(54) **Post-combustion determination of the water content of a solid fuel**

Bestimmung des Wassergehalts eines festen Brennstoffes nach seiner Verbrennung

Détermination post-combustion de la teneur en eau d'un combustible solide

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.04.2014 Bulletin 2014/18**

(73) Proprietor: **Elomatic Oy**
**20810 Turku (FI)**

(72) Inventor: **Nummila, Mika**
**40200 Jyväskylä (FI)**

(74) Representative: **Berggren Oy, Turku**
**P.O. Box 99**
**Tykistökatu 2-4 B**
**20521 Turku (FI)**

(56) References cited:
**EP-A1- 1 832 809**

- **SVEN HERMANSSON ET AL: "On-line monitoring of fuel moisture-content in biomass-fired furnaces by measuring relative humidity of the flue gases", CHEMICAL ENGINEERING RESEARCH AND DESIGN, PART A, INSTITUTION OF CHEMICAL ENGINEERS, XX, vol. 89, no. 11, 29 March 2011 (2011-03-29), pages 2470-2476, XP028307244, ISSN: 0263-8762, DOI: 10.1016/J.CHERD.2011.03.018 [retrieved on 2011-04-16]**

EP 2 725 354 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD OF THE INVENTION

The invention relates to operating combustion processes, e.g. in power plants. The invention especially relates to a new method of determining water content of a fuel for a combustion process.

### TECHNICAL BACKGROUND

Many fuels, such as wood or peat, include moisture, i.e. water. The moisture content or water content of a fuel affects its heat value. Therefore, the price of the fuel for power plants, such as thermal power stations and combined heat and power stations is based on the heat value of the fuel. Water content of the fuel fed into a combustion process may vary significantly with time. In power plants and thermal power stations the water content of the fuel is usually determined by manually analyzing samples of the fuel. The sample is weighed, dried and weighed again, the difference between the two masses indicating the amount of water in the fuel.

The manual methods are slow. There is no real possibility to notice variations in the water content with a reasonable accuracy. As a consequence, the price paid for the fuel is normally wrong. Also, the effect of the variable amount of water fed into the combustion chamber may not be adequately taken into account when operating the combustion process.

Patent publication US 6,752,093 B2 describes a refuse incineration plant, where moisture content of the flue gases is used as measure for the calorific value of the refuse. But it does not present a solution for determining water content of a fuel, let alone one that would take into account all the important factors in an adequate way.

Patent application publication EP 1 832 809 A1 discloses a real-time control of a waste incineration plant treating waste of variable moisture content. In a method for controlling a waste combustion process, the water content of the incoming waste is estimated via a parameter estimation algorithm for time varying parameters based on Kalman filters.

Article "On-line monitoring of fuel moisture-content in biomass-fired furnaces by measuring relative humidity of the flue gases" by Sven Hermansson et al. (Chemical Engineering Research and Design, vol. 89, no. 11, pages 2470-2476) discloses an indirect method for determining the moisture content of a fuel in biomass-fired furnaces by measuring relative humidity of the flue gases.

### OBJECTS OF THE INVENTION

It is an object of the present invention to reduce or even eliminate the above-mentioned problems appearing in prior art.

The present invention has an object of providing a method of determining water content of a fuel for a combustion process, which method is not based on individual samples of the fuel but a continuous measuring of the process.

An object of the present invention is to reduce the influence of variable water content, composition, grain size or temperature of the fuel on the determination of the water content of the fuel.

An object of the present invention is to allow the determination of the water content of the fuel after the combustion of the fuel.

An object of the present invention is to allow accurate determination of water content of an icy fuel.

An object of the present invention is to allow accurate and swift regulating of operating parameters of a combustion process, especially in power plants, such as thermal power stations and combined heat and power stations.

### SUMMARY OF THE INVENTION

Among others, in order to realize the objects mentioned above, method of determining water content of a fuel and other objects according to the invention are characterized by what is presented in the enclosed independent claim.

The embodiments, examples and advantages mentioned in this text relate, where applicable, to the methods according to the invention, even though it is not always specifically mentioned.

The embodiments of the invention are suitable to be used in different kinds of energy producing facilities with combustion processes. The invention is suitable for many kinds of combustion chambers, e.g. for Fluidized Bed Combustion (FBC), grate fired boilers and rotary-kiln incinerators.

The invention is especially suitable for combustion processes using only one fuel type or grade.

The embodiments of the invention are especially suitable to be used with different kinds of biofuels such as wood and peat. Other fuels, such as coal, are also suitable. Especially suitable are fuels having a relatively homogenous chemical composition, e.g. wood, peat and coal.

It has now been found that water content of a fuel can be determined with an indirect method in which the water content of the unburned fuel is not measured from the fuel itself. The idea is based on the fact that the water in the fuel will be present in flue gases, the water content of which is easily measured. Water in the flue gases originates from either:

- water in the combustion air,
- water in the fuel, or
- water from the combustion process, i.e. the chemical reaction.

According to the invention the determination of the water content of a fuel is based on measuring water content of the combustion air and water content of the flue gases. Assuming that the fuel has a chemically homogenous composition this measured data may be converted into water content of the fuel with a small number of mathematical equations presented below.

In a typical method of determining water content of a fuel for a combustion process according to the invention the water content y of a fuel is calculated from

$$y = a \cdot (\omega_{fwm} - d \cdot \omega_{am})^2 + b \cdot (\omega_{fwm} - d \cdot \omega_{am}) + c \qquad [1]$$

where $\omega_{fwm}$ is measured absolute water content of the flue gases, $\omega_{am}$ is measured absolute water content of the combustion air, and d is a coefficient for absolute water content of combustion air. d may be obtained e.g. from a database. Coefficients a, b and c are specific for a specific fuel. They may be obtained from e.g. a database.

In the invention the determination of the water content of a fuel is also based on measuring oxygen content in the flue gases.

In the invention the coefficients a, b and c are obtained from

$$a = a1 \cdot z + a2 \qquad [2]$$

$$b = b1 \cdot z + b2 \qquad [3]$$

$$c = c1 \cdot z + c2 \qquad [4]$$

where a1, a2, b1, b2, c1 and c2 are empirically determined coefficients for the specific fuel, obtained e.g. from a database. z is measured oxygen content of the flue gases in %-vol.

In the invention d is obtained from

$$d = d1 \cdot z + d2 \qquad [5]$$

where d1 and d2 are empirically determined coefficients for the specific fuel, obtained e.g. from a database. z is measured oxygen content of the flue gases in %-vol.

When the coefficients a1, a2, b1, b2, c1, c2, d1 and d2 are known, the water content of a fuel can be determined by solving simple mathematical equations. The computing power needed to solve such equations is very small. Thus e.g. a small logic circuit is enough for the computing.

The invention is easily realized and requires only minor investments. The measurements needed may be done with instruments, gauges or sensors readily available in prior art. The water content of the combustion air and the flue gases may be measured e.g. with a normal humidity sensor for gases, sold e.g. by Vaisala. An oxygen content sensor for flue gases is already normally installed in power plants for determination of air fuel ratio of the combustion process. Simple wiring or some other data-communication means are needed between the sensors and the logic. The flue gases may need a cooler before being measured. Some new piping and control valves may be required.

The coefficients a1, a2, b1, b2, c1, c2, d1 and d2 are specific for any fuel used. Usually one set of coefficients can be used for same fuel. Naturally, the fuel composition may vary significantly e.g. between different geographical sources of the fuel. Therefore, sometimes even different batches of the same fuel may require use of different coefficients. In practise, the user of the fuel, such as the power plant operator, may acquire these coefficients e.g. from a database. Also the supplier of the fuel may give the coefficients for the fuel purchased.

In an embodiment of the invention the coefficients a1, a2, b1, b2, c1, c2, d1 and d2 are determined based on combustion calculations. Each fuel has its own chemical composition and therefore specific values for the coefficients. One embodiment of such determination process for the coefficients is depicted with the following steps 1-6. All the calculations needed are known prior art as such and not discussed in more detail in this text.

Step 1: Water content of flue gas is calculated with several values for residual oxygen (02) content in flue gases and several fuel water contents. The result is a matrix of values of flue gas water content in kg/kg$_{dg}$ (kg water per kg dry gas). Relative humidity of the combustion air can be assumed as 0 %.

Step 2: A group of quadratic curves is formed from the values of the matrix in Step 1. Each quadratic curve is representing fuel water content as function of absolute water content of the flue gases with certain residual oxygen (02) content; i.e a group of curves with different residual 02 content is formed.

Step 3: A mathematical equation is formed for each individual curve in Step 2 so that group of quadratic equations is formed. The form of the equation is shown in equation [1] above.

Step 4: In the equation [1] a, b and c are varying depending on the residual 02 content of the flue gas. From each quadratic curve in Step 3 values for a, b and c can be obtained. Values for a, b and c are forming linear equations as a function of residual oxygen (02) in flue gases. The form is shown in equations [2], [3] and [4] above.

Step 5: Values a1, a2, b1, b2, c1 and c2 are obtained as constants in each linear equation obtained in Step 4.

Step 6: Values d1 and d2 represent affection of combustion air humidity in equation [5]. Values are calculated as a function of residual oxygen (02) content in flue gases.

Each fuel with a certain chemical composition has its own specific coefficients a1, a2, b1, b2, c1, c2, d1 and d2.

There is a method for operating a combustion process in which the water content of the fuel is determined and used as one of the measured variables when regulating one or more operating parameters of the combustion process. One typical method of this kind comprises at least the following steps:

- feeding a fuel and combustion air into a combustion chamber,
- producing heat and flue gases in the combustion chamber,
- transporting the flue gases out of the combustion chamber through a flue gas duct,
- regulating a plurality of operating parameters, including at least one operating parameter from the group of

  - amount of fuel fed into the combustion chamber
  - amount of combustion air fed into the combustion chamber,
  - amount and/or portions of one or more of primary, secondary and tertiary combustion air,
  - amount of circulation gas,
  - amount of chemicals to reduce NOx, i.e. nitrogen oxides in the flue gases,
  - combustion temperature, especially to reduce NOx in the flue gases, as a function of a plurality of measured variables, including at least the following measured variables:

    - absolute water content of the flue gases,
    - absolute water content of the combustion air,
    - oxygen content of the flue gases,
    - water content of the fuel.

A method for operating a combustion process of this type can be used e.g. in a power plant.

BRIEF DESCRIPTION OF THE FIGURES

The invention is described in more detail below with reference to the enclosed schematic drawing, in which

Figure 1     shows quadratic equations and values for peat water content as a function of absolute water content of the flue gases at certain residual oxygen content in flue gases,

Figure 2     shows linear equations and values for coefficients a, b and c as a function of residual oxygen content in flue gases,

Figure 3     shows an example of a method according to the invention, and

Figure 4     shows an example of method for operating a combustion process.

DETAILED DESCRIPTION OF THE EXAMPLES OF THE FIGURES

In the following we describe a calculation process with 6 steps for defining coefficients a1, a2, b1, b2, c1, c2, d1 and d2 to be used in calculations according to the invention for a certain type of Finnish peat.

Step 1: Moisture i.e. water content of flue gas is calculated with several values (2 to 5 %-vol.) for residual oxygen (02) content in flue gases and several different values (34 to 52 %) for fuel water content. The result is a matrix (Table 1) of values of flue gas water content in kg/kg$_{dg}$ (kg water per kg dry gas). Relative humidity of the combustion air can be assumed as 0 %.

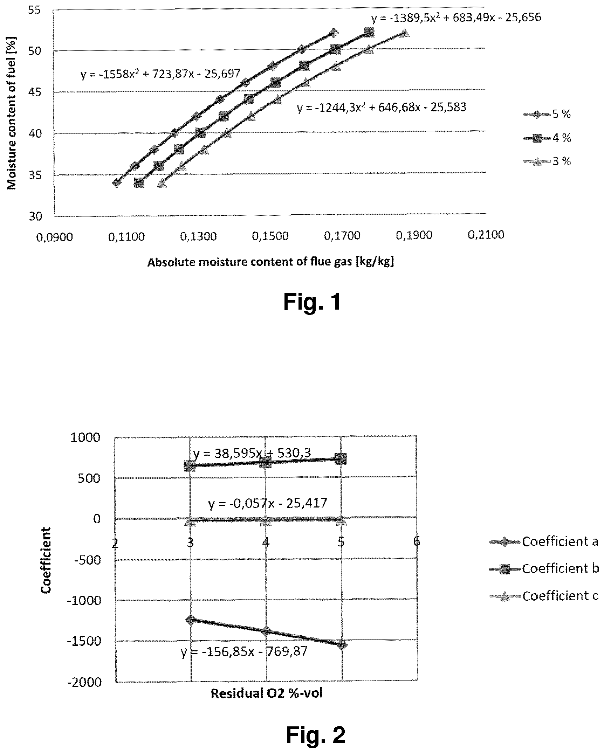

Step 2: A group of quadratic curves is formed from the values of the matrix i.e. Table 1. Each quadratic curve is representing fuel water content as function of absolute water content of the flue gas with certain residual 02 content; i.e a group of curves with different residual 02 content is formed. In Fig. 1 are shown three curves, i.e. for residual 02 content of 3 %, 4 % and 5 %.

Table 1:

| Fuel moisture content % | Water content in flue gas [kg/kg] for typical Finnish peat | | | |
|---|---|---|---|---|
| | 5,00 %-vol | 4,00 %-vol | 3,000 %-vol | 2,000 %-vol |
| 34 | 0,1074 | 0,1136 | 0,1199 | 0,1262 |
| 36 | 0,1124 | 0,1190 | 0,1255 | 0,1321 |
| 38 | 0,1178 | 0,1247 | 0,1316 | 0,1384 |
| 40 | 0,1235 | 0,1307 | 0,1380 | 0,1451 |
| 42 | 0,1297 | 0,1372 | 0,1448 | 0,1523 |
| 44 | 0,1362 | 0,1442 | 0,1521 | 0,1601 |
| 46 | 0,1433 | 0,1516 | 0,1600 | 0,1683 |
| 48 | 0,1509 | 0,1597 | 0,1685 | 0,1773 |
| 50 | 0,1591 | 0,1684 | 0,1777 | 0,1869 |
| 52 | 0,1680 | 0,1778 | 0,1876 | 0,1974 |

Step 3: A mathematical equation is formed for each individual curve from step 2 so that group of quadratic equations is formed. The form of the equation is shown in equation [1] above. The three equations for the three curves in Fig. 1 are shown in Fig. 1.

Step 4: In the equation [1] a, b and c are varying depending on the residual 02 content of the flue gas. From each quadratic curve values for a, b and c can be obtained. Values for a, b and c are forming linear equations as a function of residual oxygen (02) in flue gases. The form of these equations is shown in equations [2], [3] and [4] above. The equations for a, b and c are shown in Fig. 2.

Step 5: Values a1, a2, b1, b2, c1 and c2 are obtained as constants in each equation mentioned in Step 4 and shown in Fig. 2.

Step 6: Values d1 and d2 represent affection of combustion air humidity in equation [5]. Values are calculated as a function of residual oxygen (02) content in flue gases.

In the embodiment described above the coefficients have following values:

a1 is -156.85
a2 is -769.87
b1 is 38.595
b2 is 530.3
c1 is -0.057
c2 is -25.417
d1 is 0.0028
d2 is 0.9369.

When these coefficients are fed into equations [1] to [5] the water content of the peat used as a fuel can be calculated continuously in the power plant. Coefficients for a number of different fuels may be stored e.g. in a database or similar, from where the computer program calculating the water content y of the fuel obtains them when needed. One possibility is to input the coefficients manually to the computer program, if the chemical composition of the fuel changes.

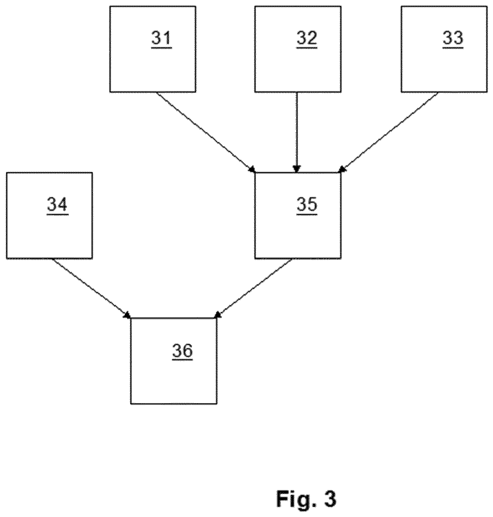

Fig. 3 shows an example of how water content of a fuel for a combustion process may be determined according to the invention. Absolute water content $\omega_{fwm}$ of flue gases from a combustion process is measured in stage 31. The gauge metering the absolute water content $\omega_{fwm}$ is situated e.g. in the flue gas duct leading the flue gases out from a combustion chamber of a power station or similar. Absolute water content $\omega_{am}$ of combustion air is measured in stage 32 with a suitable instrument, situated e.g. in the combustion air duct leading to the combustion chamber. Absolute water content of the flue gases originating from the fuel and the combustion reaction, i.e. the corrected water content $\omega_{fwc}$ is calculated in stage 35. It is obtained from equation $\omega_{fwc} = \omega_{fwm} - d \cdot \omega_{am}$, where d is coefficient for absolute water content of the combustion air. Coefficient d is specific for the fuel used and obtained e.g. from a database in stage 33. The water content y of the fuel is calculated in stage 36 from $y= a\cdot\omega_{fwc}{}^2 + b\cdot\omega_{fwc} + c$ or as it is written in equation [1]: $y= a\cdot(\omega_{fwm} - d\cdot\omega_{am})^2 + b\cdot(\omega_{fwm} - d\cdot\omega_{am}) + c$. Coefficients a, b and c are specific for the fuel used and obtained e.g. from a database in stage 34.

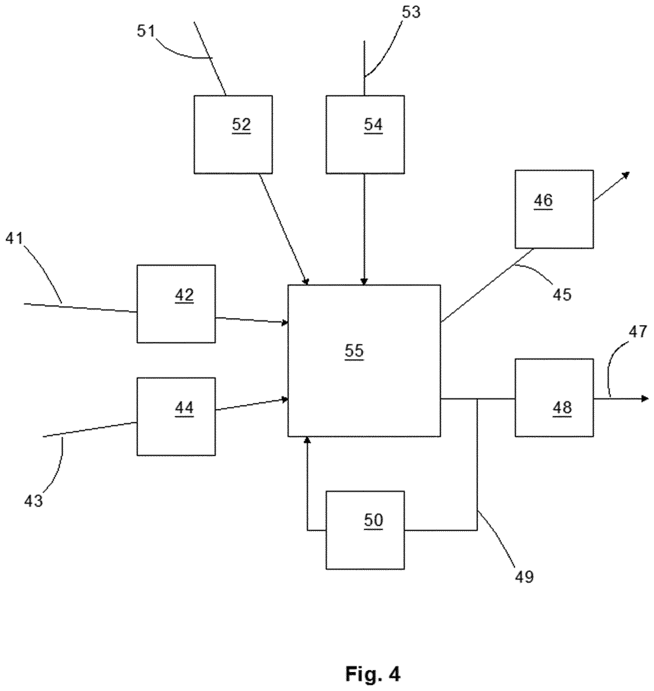

Fig. 4 shows an example of a method for operating a combustion process. Arrow 41 illustrates the fuel fed into the combustion process, i.e. into combustion chamber 55. Arrow 43 illustrates combustion air, 45 is the energy produced by the combustion. Arrow 47 depicts the flue gases. Arrow 49 describes how part of the flue gases are recirculated into the combustion chamber as circulation gas. Arrow 51 describes the chemicals for reducing NOx from the combustion gases. Arrow 53 depicts the various means for affecting to the temperature in the combustion chamber. Boxes on the arrows describe means and devices, such as instruments, gauges, meters, indicators and valves for measuring and controlling the parameters of the arrows. Box 42 indicates e.g. the regulator for the fuel flow into the combustion process and the instrument metering the amount of fuel fed into the combustion chamber. Box 44 indicates instruments to measure and regulate e.g. amount of combustion air fed into the combustion chamber, and possibly amount and/or portions of one or more of primary, secondary and tertiary combustion air. Box 44 also includes instrument for metering absolute water content of the combustion air. Box 46 depicts instruments measuring the amount of energy produced in the combustion chamber 55. Box 48 depicts instruments measuring absolute water content and oxygen content of the flue gases. Box 50 indicates instruments to measure and regulate the amount of circulation gas. Box 52 indicates instruments to regulate the amount of chemicals fed to the combustion process to reduce NOx in the flue gases. Box 54 indicates instruments to measure and regulate the temperature in the combustion chamber.

The method depicted in Fig. 4 functions in the following way. Fuel and combustion air are fed into the combustion chamber 55 at 41 and 43 respectively. Heat 45 and flue gases are produced. The flue gases are transported out of the combustion chamber 55 through a flue gas duct at 47. A plurality of measured variables are measured, including absolute water content of the flue gases at box 48, absolute water content of the combustion air at box 44, oxygen content of the flue gases at box 48.

A plurality of operating parameters are regulated as a function of the measured variables. The operating parameters include amount of fuel fed into the combustion chamber at box 42, amount of combustion air fed into the combustion chamber at box 44, amount and/or portions of one or more of primary, secondary and tertiary combustion air at box 44, amount of circulation gas at box 50, amount of chemicals to reduce NOx in the flue gases at box 52 and combustion temperature at box 54.

One of the measured variables in the method depicted in Fig. 4 is the water content of the fuel, which is determined according to the invention, e.g. in the way explained in Fig. 3.

The figures show only a few preferred embodiments according to the invention. Facts of secondary importance with regards to the main idea of the invention, facts known as such or evident for a person skilled in the art are not necessarily separately shown in the figures. It is apparent to a person skilled in the art that the invention is not limited exclusively to the examples described above, but that the invention can vary within the scope of the claims presented below.

## Claims

1. A method of determining water content of a fuel for a combustion process, the method comprising at least the following steps:

    - measuring (31) absolute water content $\omega_{fwm}$ of flue gases from the combustion process,
    - measuring (32) absolute water content $\omega_{am}$ of combustion air,
    - measuring oxygen content z of the flue gases from the combustion process in %-vol,

    **characterized in** calculating (36) the water content y of the fuel from

$$y= a\cdot(\omega_{fwm} - d\cdot\omega_{am})^2 + b\cdot(\omega_{fwm} - d\cdot\omega_{am}) + c$$

    where a, b and c are coefficients for a specific fuel, and d is coefficient for absolute water content of the combustion air, and wherein a, b, c and d are obtained from

$$a=a1\cdot z + a2$$

$$b = b1 \cdot z + b2$$

$$c = c1 \cdot z + c2$$

$$d = d1 \cdot z + d2$$

where a1, a2, b1, b2, c1, c2, d1 and d2 are empirically determined coefficients for the fuel, or wherein the coefficients a, b and c are determined based on combustion calculations with at least the following steps:

- creating a matrix of values of flue gas water content as a function of several values for oxygen content z in flue gases and several fuel water contents y;
- forming a group of quadratic curves from the values of the matrix, where each quadratic curve is representing fuel water content y as a function of absolute corrected water content $\omega_{fwc}$ of the flue gases with certain oxygen content z in the flue gases, where $\omega_{fwc} = \omega_{fwm} - d \cdot \omega_{am}$, i.e. a group of quadratic curves each with a different residual oxygen content of the flue gas is formed;
- forming a mathematical equation in form $y = a \cdot (\omega_{fwc})^2 + b \cdot (\omega_{fwc}) + c$ for each said quadratic curve;
- obtaining values for a, b and c for each said quadratic curve.

**Patentansprüche**

1. Verfahren zum Bestimmen eines Wassergehalts eines Kraftstoffs für einen Verbrennungsvorgang, wobei das Verfahren zumindest die folgenden Schritte umfasst:

- Messen (31) eines absoluten Wassergehalts $\omega_{fwm}$ von Abgasen von dem Verbrennungsvorgang,
- Messen (32) eines absoluten Wassergehalts $\omega_{am}$ von Verbrennungsluft,
- Messen eines Sauerstoffgehalts z der Abgase von dem Verbrennungsvorgang in Volumenprozenten,

**gekennzeichnet durch** Berechnen (36) des Wassergehalts y des Kraftstoffs aus

$$y = a \cdot (\omega_{fwm} - d \cdot \omega_{am})^2 + b \cdot (\omega_{fwm} - d \cdot \omega_{am}) + c$$

wobei a, b und c Koeffizienten für einen speziellen Kraftstoff sind und d ein Koeffizient für einen absoluten Wassergehalt der Verbrennungsluft ist, und wobei a, b, c und d erhalten werden aus

$$a = a1 \cdot z + a2$$

$$b = b1 \cdot z + b2$$

$$c = c1 \cdot z + c2$$

$$d = d1 \cdot z + d2$$

wobei a1, a2, b1, b2, c1, c2, d1 und d2 empirisch bestimmte Koeffizienten für den Kraftstoff sind, oder wobei die Koeffizienten a, b und c basierend auf Verbrennungsberechnungen mit mindestens den folgenden Schritten bestimmt werden:

- Erzeugen einer Matrix von Werten von Abgaswassergehalt als eine Funktion von mehreren Werten für Sauerstoffgehalt z in Abgasen und mehreren Kraftstoffwassergehalten y;

- Bilden einer Gruppe von Kurven zweiter Ordnung aus den Werten der Matrix, wobei jede Kurve zweiter Ordnung einen Kraftstoffwassergehalt y als eine Funktion eines absoluten korrigierten Wassergehalts $\omega_{fwc}$ von den Abgasen mit einem bestimmten Sauerstoffgehalt z in den Abgasen darstellt, wobei $\omega_{fwc} = \omega_{fwm} - d \cdot \omega_{am}$ gilt, d.h. eine Gruppe von Kurven zweiter Ordnung mit jeweils einem unterschiedlichen Restsauerstoffgehalt des Abgases wird gebildet;
- Bilden einer mathematischen Gleichung in der Form $y = a \cdot (\omega_{fwc})^2 + b \cdot (\omega_{fwc}) + c$ für jede der Kurven zweiter Ordnung;
- Erhalten von Werten für a, b und c für jede der Kurven zweiter Ordnung.

**Revendications**

1. Procédé de détermination de la teneur en eau d'un combustible pour un processus de combustion, le procédé comprenant au moins les étapes suivantes :

   - la mesure (31) de la teneur en eau absolue $\omega_{fwm}$ de gaz de carneau provenant du processus de combustion,
   - la mesure (32) de la teneur en eau absolue $\omega_{am}$ de l'air de combustion,
   - la mesure de la teneur en oxygène z des gaz de carneau provenant du processus de combustion en % en volume,

   **caractérisé par** le calcul (36) de la teneur en eau y du combustible à partir de

   $$y = a \cdot (\omega_{fwm} - d \cdot \omega_{am})^2 + b \cdot (\omega_{fwm} - d \cdot \omega_{am}) + c$$

   où a, b et c sont des coefficients pour un combustible spécifique, et d est le coefficient pour la teneur en eau absolue de l'air de combustion, et dans lequel a, b, c et d sont obtenus à partir de

   $$a = a1 \cdot z + a2$$

   $$b = b1 \cdot z + b2$$

   $$c = c1 \cdot z + c2$$

   $$d = d1 \cdot z + d2$$

   où a1, a2, b1, b2, c1, c2, d1 et d2 sont des coefficients déterminés de façon empirique pour le combustible, ou dans lequel les coefficients a, b et c sont déterminés en fonction de calculs de combustion avec au moins les étapes suivantes :

   - la création d'une matrice de valeurs de la teneur en eau de gaz de carneau en fonction de plusieurs valeurs pour la teneur en oxygène z dans les gaz de carneau et de plusieurs teneurs en eau y du combustible ;
   - la formation d'un groupe de courbes quadratiques à partir des valeurs de la matrice, où chaque courbe quadratique représente la teneur en eau y du combustible en fonction d'une teneur en eau corrigée absolue $\omega_{fwc}$ des gaz de carneau avec une certaine teneur en oxygène z dans les gaz de carneau, où $\omega_{fwc} = \omega_{fwm} - d \cdot \omega_{am}$, c'est-à-dire qu'un groupe de courbes quadratiques ayant chacune une teneur en oxygène résiduelle différente du gaz de carneau est formé ;
   - la formation d'une équation mathématique sous la forme $y = a \cdot (\omega_{fwc})^2 + b \cdot (\omega_{fwc}) + c$ pour chaque dite courbe quadratique ;
   - l'obtention de valeurs pour a, b et c pour chaque dite courbe quadratique.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6752093 B2

- EP 1832809 A1

**Non-patent literature cited in the description**

- **SVEN HERMANSSON et al.** On-line monitoring of fuel moisture-content in biomass-fired furnaces by measuring relative humidity of the flue gases. *Chemical Engineering Research and Design,* vol. 89 (11), 2470-2476